# EUROPEAN PATENT SPECIFICATION

(11) **EP 1 806 109 B1**
(45) Date of publication and mention of the grant of the patent: **16.07.2008**
(21) Application number: 06000385.2
(22) Date of filing: 10.01.2006
(51) Int. Cl.: A61B 19/00

(54) **Apparatus for femur head centre localization**
Apparat zur Bestimmung des Ortes des Femurkopfes
Appareil pour la localisation de la tête fémorale

(43) Date of publication of application: 11.07.2007
(73) Proprietor: BrainLAB AG, 85551 Kirchheim/Heimstetten (DE)
(72) Inventor: Götte, Hubert, 80333 München (DE); Immerz, Martin, 82166 Gräfelfing (DE)
(74) Representative: Schwabe - Sandmair - Marx

(56) References cited:
- WO-A-20/05053559

## Description

The present invention concerns an apparatus for determining the femur head centre location without a femur marker array.

Usually a femur marker array and a tibia marker array are used to determine the position of the femur, especially the femur head centre and the tibia, if surgical procedures at the knee are conducted.

WO 2005/053559 A1 discloses an apparatus for providing a navigational array which can be used to track particular locations associated with various body parts such as tibia and femur to which reference arrays are implanted. A position sensor can sense data relating to the position and orientation of the reference arrays. In a prosthetic installation procedure, a surgeon can designate a center of rotation of a patient's femoral head for purposes of establishing the mechanical axis and other relevant constructs relating to the patient's femur according to which prosthetic components can ultimately be positioned. Such center of rotation can be established by articulating the femur within the acetabulum or a prosthesis to capture a number of samples of position and orientation information and thus in turn to allow the computer to calculate the average center of rotation.

It is an object of the invention to determine or define the location of the femur head centre while improving the accessibility of the knee joint and to reduce invasiveness.

This object is solved by an apparatus according to claim 1.

According to the invention, the location of the femur head centre is determined by using a tibia marker array only which can also be used for subsequent navigation purposes on tibia or femur.

A three-step approach including calibration, attachment and reproduction can be used for the present invention.

### Calibration

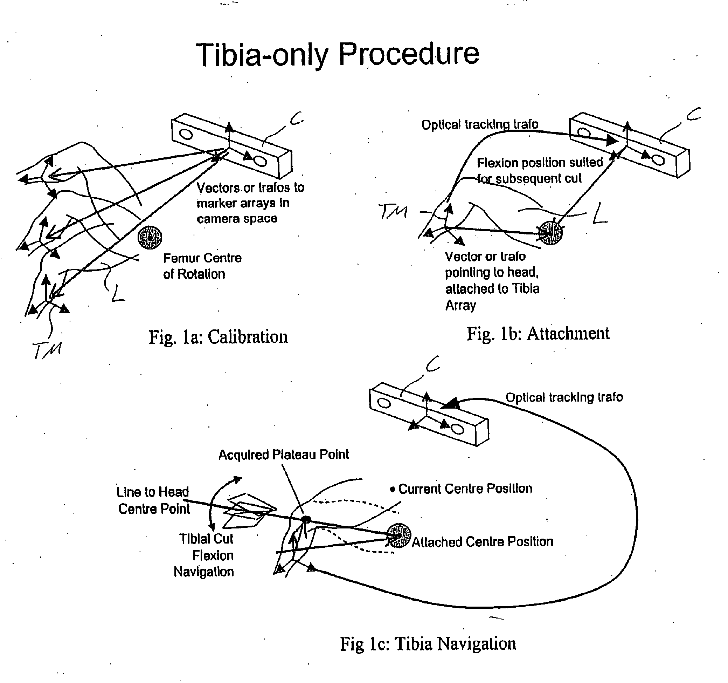

As shown in Figure 1a, a kinematical model of the leg including the femur centre of rotation is determined by using a tibia marker array TM attached to the patient's leg L and moving the leg L to different positions during a calibration procedure. The marker array TM can be either fixed directly to the tibia or can be fixed to the leg using e.g. a Velcro ® without performing any surgical steps.

### Attachment

The determined femur centre of rotation position is virtually connected to the tibia marker array TM to describe its position for a specific user-defined position of the patient's leg, as e.g. for a specific flexion as shown in Figure 1b. This can be sufficient for navigated surgical steps on the tibia alone, which just rely on the femur head position in a specific knee position, as described below as "tibia-only workflow". For example, the proximal tibia cut could be aligned to the femur mechanical axis established in 90° flexion of the knee joint.

### Reproduction

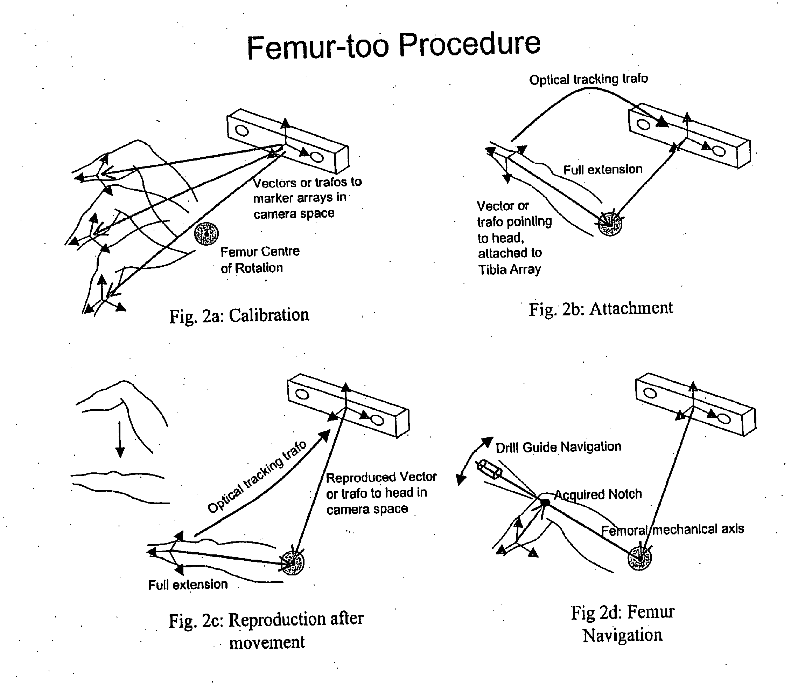

For later reproduction after patient movements, the initially found centre position is transformed to camera space by reproducing the initially user-defined leg position and capturing the corresponding tibia marker position with the camera system, as shown in Figure 2c.

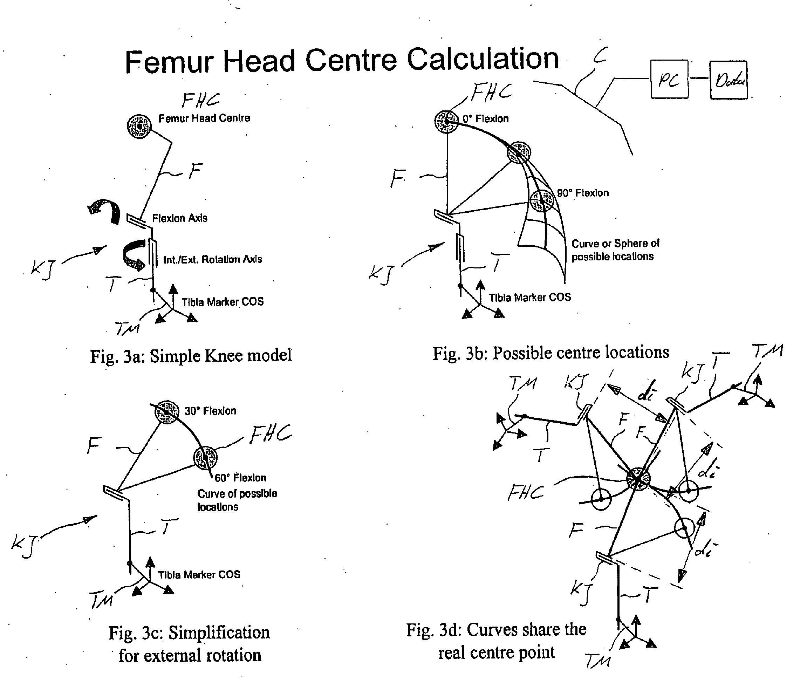

The invention simplifies the knee joint kinematics to a mechanical model with few, e.g. two or in a specific defined position of the tibia relative to the knee or the femur only one fixed rotational degrees of freedom. A possible concept amongst others is a model with two rotational degree of freedom, as shown in Figure 3a. A hinge is being used to describe knee flexion and another one is being used to describe tibia rotation within the knee joint KJ. The femur head centre FHC sits at the end of a link attached to the flexion axis. The tibia marker array TM sits at the end of a link attached to the rotation axis. These rotational axes form a simplified mechanical model of the knee joint KJ. Their positions and orientations with respect to each other and the marker array TM are the mechanical parameters of the model. In the simplest possible example configuration, both rotational axes are orthogonal and the femoral head centre FHC moves on a regular sphere with respect to the tibia T, as shown in Figures 3a and b.

For a specific patient with a marker array TM attached to the tibia T in a specific position, the model parameters are unknown before calibration. After calibration they can be calculated.

### Calibration

Calibration is carried out with rotational and translational movements of the tibia T and the femur F around the femur head centre FHC located in the pelvis, as shown in Figure 1a. The centre point itself is kept still in space while the leg is being moved and the knee is being bent during the calibration run.

The orientations and the locations of the two rotational axes of the knee joint hinges are being derived from a data set of positions of the tibia array acquired with the camera system. Furthermore, the location of the femur head centre is being calculated with respect to the flexion hinge. With these parameters, the mechanical model is defined and can describe the possible locations of the femoral head centre FHC in dependency to the current flexion and internal rotation angles applied to the hinges.

The calibration procedure utilizes the fact, that the parameters of the model except for the flexion and rotation angles must be the same for all acquired tibia positions during the calibration run. Furthermore, the femur head centre position with respect to the camera coordinate system keeps still during the tibia movements. If the mechanical model is applied to describe the possible femur head centre points for all of the recorded tibia array positions, there must be a common point in camera space contained by all of the models. This common point in camera spare is the femur head centre point FHC, as shown in Figure 3d. The calibration algorithm varies the mechanical parameters to establish this common point with minimum error. Thus, a distance di (or "a" according to the Denavit-Hartenberg notation) of the femur head centre FHC from the simplified knee joint KJ is calculated so that a single point of intersection is found. For distances larger or smaller than di there could be more points of intersection.

In general the knee or one or more joint elements of a body can be modelled as a kinematical chain which can be moved to determine the parameters describing the model to obtain finally the location of the centre of rotation of one end element of the chain being kept fixed while using and tracking the movements of only a single marker or reference array connected to an opposite end element of the kinematical chain.

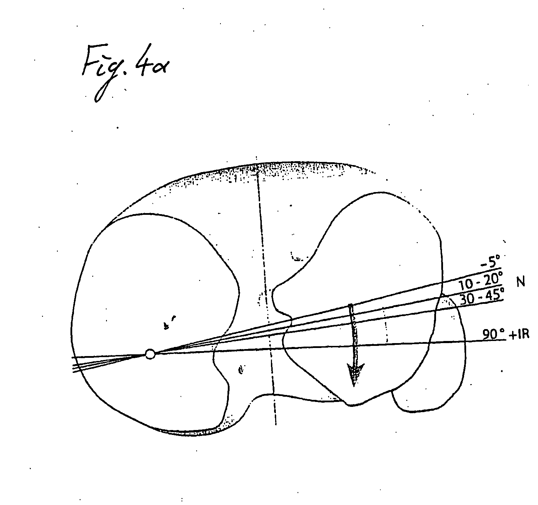

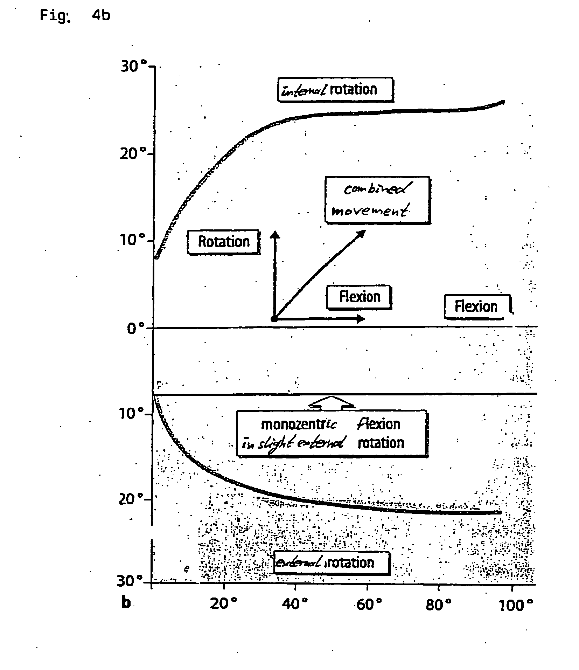

Biomechanical literature describing the behaviour of the physiological knee joint support the idea of a hinge kinematic under certain circumstances. Hassenpflug J: "Gekoppelte Knieendoprothesen" describes in Der Orthopäde 6 (2003) 32, S.484-489 that under external rotation the orientation of the flexion axis keeps fixed over a certain flexion range (mono-centric behaviour). Thus the knee joint degenerates to a single flexion hinge (external rotation stays fixed to a constant value), see Fig. 4a and 4b. Wetz H. et al.: "Die Bedeutung des dreidimensionalen Bewegungsablaufes des Femurotibialgelenks für die Ausrichtung von Knieführungsorthesen" in Der Orthopäde 4 (2001) 30, S. 196-207 supports the idea of simplifying knee kinematics to a flexion hinge in the flexion range of about 25° to 90° with his own findings about the location of the knee axes.

The reported physiological behaviour can be used to simplify the mechanical model within the invention even further by skipping the second hinge used for internal respective external rotation, see Figure 3c. To achieve this, the tibia can be rotated to a specific location or position where further rotation of the tibia T is restricted or limited wherein the tibia is held in this position to relative to the femur or knee during further movement of the leg. For maximum computing stability, calibration should then be conducted in the range of 30 to 90° flexion and concomitant maximum external respective internal rotation by the surgeon.

### Attachment

After calibration, the femur head centre location is defined within the kinematical model. Its position and orientation with respect to the tibia marker array TM is then computed for the user-defined current stance and virtually attached by means of a calculated transformation matrix to the tibia marker array TM, see Figures 1b and 2b. This transformation is valid for the current stance. It can now be exploited for alignment purposes on the tibia, as described below in Example I.

To enable later reproduction, the initial stance should be one with a mechanically reproducible femur centre position with respect to the tibia, as e.g. full extension paired with high external rotation, as described below. Thus, it keeps valid with respect to the tibia array despite of any camera or patient movements.

Hassenpflug 1. c. shows, that the knee joint has a certain freedom for internal respective external rotation dependent on the current flexion angle, see Fig. 4a and 4b. This freedom is minimized in full extension to a range of +/-8°. Attachment can thus be carried out e.g. in full extension and maximum external rotation (8°) to exploit this point of limit-stop as a reproducible stance. Given that no intermediate surgical steps have changed the kinematics of the joint, this stance can be reapplied at any time.

### Reproduction

Surgical steps on the femur rely on the current femur head centre position with respect to camera space. Before such a surgical step is being navigated, the femur head centre must be reproduced in camera space, see Figure 2c. After having positioned the leg in the reproducible stance, the position of the tibia marker array TM is being read form the camera system C and the known transformation matrix is being applied to calculate the current centre position in camera space. As long as the patient's hip is not being moved, the femoral head centre FHC can be used for navigation. Since typical navigation steps, as e.g. aligning a drill guide, can be carried out rather quick, the hip centre can be kept still for such short periods.

Thus, according to the invention, a femur marker array can be omitted to minimize trauma on the femur and to improve accessibility of the limited space within the knee joint during surgery, which is particularly useful for minimal invasive or time-critical surgical procedures.

Avoiding a femur marker is highly valuable for minimal invasive surgical procedures such as uni-compartmental knee procedures, where a marker array on the femur cannot be attached because of limited space or time.

Although the precision of the described approach can be limited, i.e. by the quality of the mechanical knee model used for calibration, it is beneficial for procedures where less precision for the femur head is sufficient and at the same time the application of a femoral marker array is not possible or desired. Such conditions apply to specific surgical procedures, e.g. for the Oxford uni-compartmental implant family due to its spherical constructions and the minimally invasive nature of the procedure.

The use of the invention will be described with reference to the drawings which show in
- Figures 1a to 1c: the calibration, attachment and tibia navigation in a tibia-only procedure;
- Figures 2a to 2d: the calibration, attachment, reproduction after movement and femur navigation of a femur-too procedure;
- Figures 3a to 3b: the calculation of the femur head centre according to the present invention;
- Figures 4a and 4b: the rotational behaviour of the knee joint according to Hassenpflug; and
- Figures 5a and 5b: models of the knee having one and two degrees of freedom, respectively.

### Example I

A tibia-only workflow for unicompartmental surgery using the present invention is described with reference to Figure 1.

Two tibial cuts are applied without navigating any femur surgical steps, wherein the alignment of these tibial cuts depends on the position of the femur head centre in 90° knee flexion. According to the invention, this alignment can be achieved without using a femoral marker array and without time consuming femoral registration.

After moving the knee during the calibration run, the calculated femur head centre is "attached" to the tibia maker array in a fixed position, as e.g. a 90° flexion position, and relaxed external rotation state of the knee.

The flexion angle can be adjusted to 90° before attaching the femur head centre point. This can be supported by navigation without using a femoral marker array by simply connecting a line from the known femur head centre point to the femoral notch. This point can be acquired with a pointer with the knee flexed in approximately 90° flexion. It is virtually attached to the tibia array to be tracked on further movements. When the knee is brought in such a position, that the line is orthogonal to the known tibia mechanical axis, the amount of flexion is nearly 90°. In this state, the position of the femur head centre defined in camera space is being virtually attached to the tibia marker array and the tibia cuts are subsequently being navigated.

This 90° flexion position is well suited especially for the subsequent vertical tibia cut, because it has to point to the femur head in 90° flexion of the knee. The cut can be subsequently navigated despite any simultaneous camera or patient movement, because the relevant femur centre point is virtually attached to the tibia marker array.

### Example II

A femur-too workflow in Oxford unicompartmental surgery using the present invention is described with reference to Figure 2.

Besides tibia cuts also femur cuts are being performed. A femoral drill guide is navigated to geometrically define the location of the femur implant.

The rotational alignment of the drill guide is defined in Varus-Valgus and in Flexion-Extension with respect to the femoral mechanical axis, which is defined by the femur head centre point and a notch point on the proximal femur. With the invention, the drill guide alignment can be achieved without using a femoral marker array and without femoral registration.

After calibration, the calculated femur head centre is attached to the tibia marker array after calibration in full extension and maximum external rotation applied by the surgeon. This leg position is reproducible, because any rotational freedom of the knee is locked. From this point on, surgical steps causing movements of the patient or the leg may occur. Just before the drill guide shall be navigated, the full extension stance is being re-applied to the knee by the surgeon and the tibia marker array is being captured by the camera. Then the femur head centre position defined with respect to the tibia array is transformed into camera space. Subsequent navigation of the drill guide is done in camera space with respect to the known femur head centre and the tracked tibia marker array. The leg can be brought into any convenient position for the drill guide navigation step as long as the femur head is being kept in a fixed position. Note, that unlike to the tibia-only-workflow described in Example I, any camera moves must be impeded for the time of the drill guide navigation.

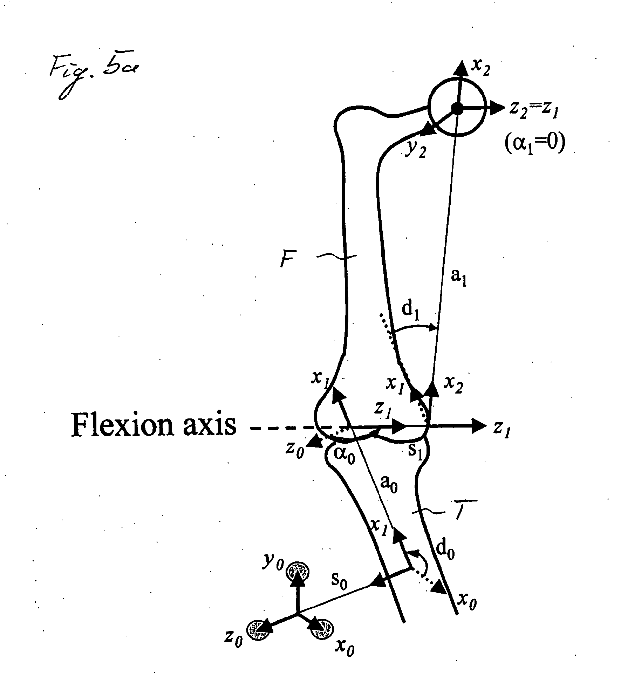

Figure 5a shows a model of a knee joint having one degree of freedom.

A single or primitive joint element is a basic or elementary joint and can e.g. be described according to the notation of Denavit-Hartenberg by the parameters s, a, α and d, wherein s and a represent translations and α and d represent a rotation.

The reference array attached to the tibia T is represented by a coordinate system 0 with the axes x₀, y₀ and z₀. The parameters s₀, d₀, a₀, α₀, s₁, d₁, a₁ and α₁ describe the geometric model, wherein parameter d₁ represents the flexion of the knee joint.

The translation of the coordinate system 0 along its z-axis z₀ by the amount of s₀, the subsequent rotation around z₀ by d₀, the subsequent translation by a₀ along the now rotated x-axis and the subsequent rotation around the rotated x-axis by α₀ yields coordinate system 1 with the coordinate axes x₁, y₁ and z₁.

Translation of coordinate system 1 along z₁ by amount s₁, subsequent rotation around z₁ by d₁, subsequent translation by a₁ along the now rotated x-axis, subsequent rotation around the rotated x-axis by α₁ yields coordinate system 2 with the axes x₂, y₂, z₂.

The origin of coordinate system 2 sits in the centre of rotation inside the femur head.

The acquisition of marker positions is a prerequisite to determine the model parameters and is performed as follows:
1. Extend the knee fully and apply maximum internal respectively external rotation to the knee in order to lock rotation. With the tibia reference array attached, conduct circular movements around the femur centre of rotation.
2. Allow flexion in the knee joint up to 30° to 40° and repeat step 1 several times with changed flexion.
3. Vary adduction respectively abduction in the hip joint and repeat step 2 several times with changed adduction respectively abduction. Always keep the rotation of the knee joint locked.

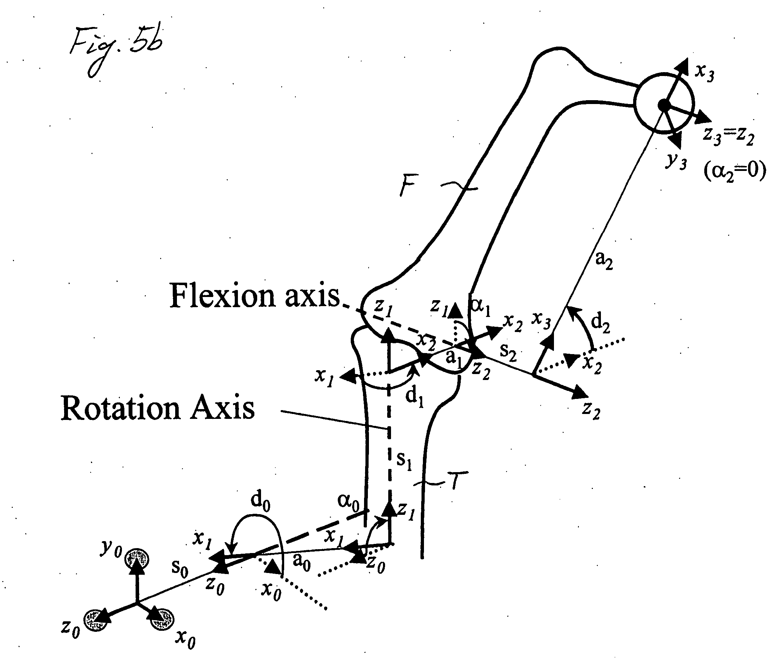

Figure 5b shows a model of the knee having two degrees of freedom.

As for figure 5a, the reference array attached to the tibia is represented by a coordinate system 0 with the axes x₀, y₀ and z₀.

The translation of coordinate system 0 along its z-axis z₀ by amount s₀, subsequent rotation around z₀, by d₀, subsequent translation by a₀ along the now rotated x-axis and subsequent rotation around the rotated x-axis by α₀ yields coordinate system 1 with the axes x₁, y₁ and z₁.

The translation of coordinate system 1 along z₁ by amount s₁, subsequent rotation around z₁ by d₁, subsequent translation by a₁ along the now rotated x-axis, and subsequent rotation around the rotated x-axis by α₁ yields coordinate system 2 with the axes x₂, y₂, and z₂.

The translation of coordinate system 2 along z₂ by amount s₂, subsequent rotation around z₂ by d₂, subsequent translation by a₂ along the now rotated x-axis, subsequent rotation around the rotated x-axis by α₂ yields coordinate system 3 with the axes x₃, y₃ and z₃.

The origin of coordinate system 3 sits in the centre of rotation inside the femur head. The parameters s₀, d₀, a₀, α₀, s₁, d₁, a₁, α₁, s₂, d₂, a₂ and α₂ describe the geometric model. Parameter d₁ represents the internal respectively external rotation and parameter d₂ the flexion of the knee joint.

To model the complex behaviour of the knee joint more adequately and in order to gain precision, it may be enhanced by introducing further sets of s, d, a, and α parameters for further degrees of freedom.

The acquisition of marker positions as prerequisite to determine the model parameters is performed as follows:
1. Extend the knee fully and apply maximum internal respectively external rotation to the knee in order to lock rotation. With the tibia reference array attached, conduct circular movements around the femur centre of rotation.
2. Allow flexion in the knee joint up to 30° to 40° and repeat step 1 several times with changed flexion. Release the locked rotation and constantly change the rotation within its physiological range.
3. Vary adduction respectively abduction in the hip joint and repeat step 2 several times with changed adduction respectively abduction.

## Claims

1. Apparatus for localizing the femur head centre (FHC) using only a tibia marker array (TM) connected to the tibia (T), comprising a camera (C) for localizing the tibia marker array (TM), a computer (PC) connected to the camera (C) to obtain the positional data of the tibia marker array (TM) from the camera images of the tibia marker array (TM) and a database (Data) storing a kinematic model of the knee joint (KJ) having at least-one or two degrees of freedom, which database (Data) is connected to the computer (PC) which calculates a distance di so that for all said positional data the lines of movement of a point (FHC) having a distance di from the knee joint (KJ) or from the joint element closest to the femur head centre (FHC) in the kinematical chain modelling the knee joint coincide in a single point which is considered to be the femur head centre (FHC).

## Patentansprüche

1. Vorrichtung zum Lokalisieren des Oberschenkelknochen-Kopfzentrums (FHC) unter Verwendung lediglich einer Schienbeinmarkierungsanordnung (TM), die mit dem Schienbein (T) verbunden ist, wobei die Vorrichtung eine Kamera (C), um die Schienbeinmarkierungsanordnung (TM) zu lokalisieren, und einen Computer (PC) umfasst, der mit der Kamera (C) verbunden ist, um die Positionsdaten der Schienbeinmarkierungsanordnung (TM) anhand der Kamerabilder der Schienbeinmarkierungsanordnung (TM) und einer Datenbank (Data) zum Speichern eines kinematischen Modells des Kniegelenks (KJ) mit wenigstens einem oder mit zwei Freiheitsgraden zu erhalten, wobei die Datenbank (Data) mit dem Computer (PC) verbunden ist, der einen Abstand di berechnet, so dass für alle Positionsdaten die Bewegungslinien eines Punkts (FHC), der einen Abstand di von dem Kniegelenk (KJ) oder von dem Gelenkelement, das sich am Nächsten bei dem Oberschenkelknochen-Kopfzentrum (FHC) in der das Kniegelenk modellierenden kinematischen Kette befindet, hat, an einem einzigen Punkt zusammentreffen, der als das Oberschenkelknochen-Kopfzentrum (FHC) angesehen wird.

## Revendications

1. Dispositif pour localiser le centre de la tête fémorale (FHC) en utilisant uniquement un réseau de marqueurs de tibia (TM) relié au tibia (T), comportant un appareil de prise de vues (C) pour localiser le réseau de marqueurs de tibia (TM), un ordinateur (PC) relié à l'appareil de prise de vues (C) pour obtenir les données positionnelles du réseau de marqueurs de tibia (TM) à partir des images de l'appareil de prise de vues du réseau de marqueurs de tibia (TM) et d'une base de données (Data) mémorisant un modèle cinématique de l'articulation du genou (KJ) ayant au moins un ou deux degrés de liberté, laquelle base de données (Data) est reliée à l'ordinateur (PC) qui calcule une distance di de sorte que, pour la totalité desdites données positionnelles, les lignes de déplacement d'un point (FHC) ayant une distance di par rapport à l'articulation du genou (KJ) ou par rapport à l'élément d'articulation le plus proche du centre de la tête fémorale (FHC) dans la chaîne cinématique modélisant l'articulation du genou, coïncident en un point unique qui est considéré être le centre de la tête fémorale (FHC).
